# EUROPEAN PATENT APPLICATION

(11) **EP 1 475 378 A1**
(43) Date of publication of application: **10.11.2004**
(21) Application number: 04013362.1
(22) Date of filing: 14.12.2000
(51) Int. Cl.: C07D 417/12, A61K 31/4439, A61P 3/10

(54) **Antidiabetic thiazolidinedione potassium salt**

(30) Priority: 18.12.1999 HU 9904634
(62) Divisional of application: 00985704.6
(71) Applicant: RICHTER GEDEON VEGYESZETI GYAR R.T., 1103 Budapest (HU)
(72) Inventor: The designation of the inventor has not yet been filed
(74) Representative: Bublak, Wolfgang, Dr.

(57) **Abstract**

The invention relates to a compound of formula (I) where the meaning of X⁺ is potassium cation, and to its use as antidiabetic.

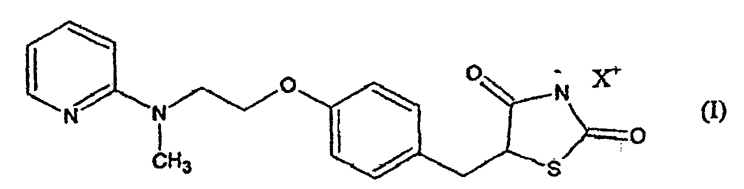

## Description

This invention relates to a new process for the synthesis of the compounds of the general formula (I) 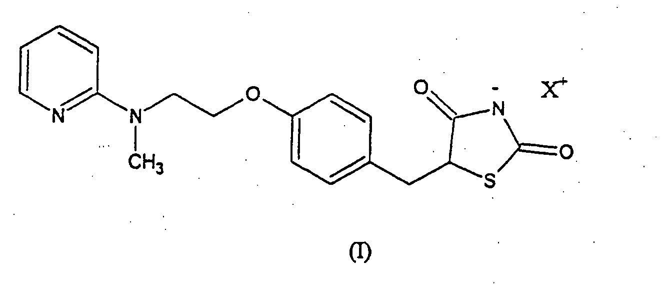 through the new intermediates of the general formula (II), 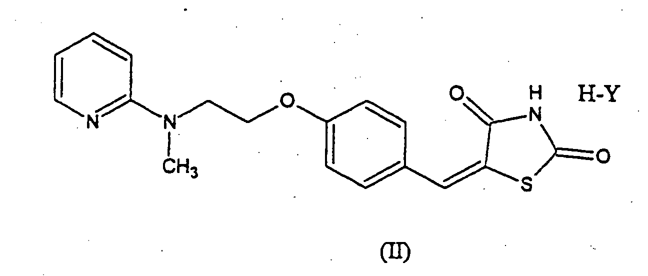 where the meaning of X⁺ in the formula is alkali metal or alkaline-earth metal cation; HY means hydrogenchloride acid, p-toluenesulfonic acid, formic acid or trifluoroacetic acid.

The 2,4-thiazolidinedione salts of general formula (I) are the salt derivatives of rosiglitazone, which is a known drug for the treatment of the noninsulin-dependent diabetes. The chemical name of rosiglitazone is:
5-{4-[2-(N-methyl-N-(2-pyridyl)-amino)-ethoxy]-benzyl}-2,4-thiazolidinedione.

The rosiglitazone, its derivatives and their synthetic procedures are disclosed in EP 306228 but the rosiglitazone salts are not described in it.

The salts of the rosiglitazone and its derivatives with acids of H⁺M⁻ formula are described in PCT WO 94/05659 where the meaning of the M⁻ in the formula is a counter-ion, preferably maleate ion.

The PCT WO 98/39006 mentions salts of thiazolidine derivatives with acids and bases but their syntheses were not described.

The EP 306228 describes the synthetic process of the 5-{4-[2-(N-methyl-N-(2-pyridyl)-amino)-ethoxy]-benzylidene}-2,4-thiazolidinedione base of the formula (III). According to our experiments this procedure does not afford a pure product: the melting range was between 177-190°C, which correlates to a mixture. Based on our HPLC measurements the mixture contained not more than 70-80% of the product.

According to the PCT WO 94/05659 rosiglitazone maleate can be synthesised from rosiglitazone base in a yield of 73% only. Based on our experiments the crystallisation of the product is very difficult because it is susceptible to precipitate as an oil and during its crystallisation impurities appear in the product in an undesirable measure. According to our HPLC measurements the product contained 0.41% total impurity where the quantity of the individual impurity components amounted to 0.15%.

For this reason our aim was to develope a new, technologically better procedure. In the course of our experiments we surprisingly found that the base of formula (III) 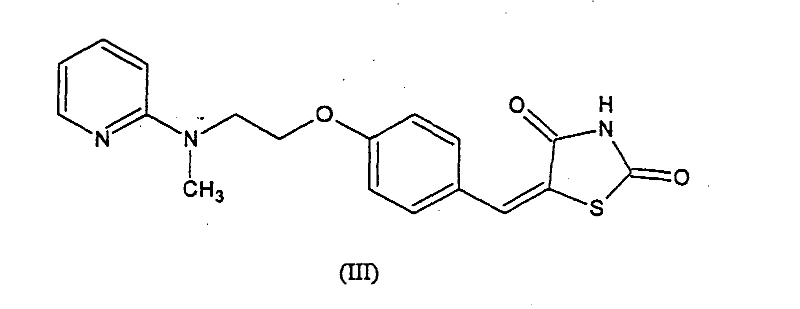 can be purified in an excellent way as a salt form what was generated with an acid of pK < 4. The effectivity of the purification is very good in the case of the trifluoroacetate salt affording a product with a melting point of 194-196°C, and according to our HPLC measurements its purity is 99,5%.

The compounds of the general formula (II), their tautomer forms and their solvates the name of the base-component is: 5-{4-[2-(N-methyl-N-(2-pyridyl)-amino)-ethoxy]-benzylidene}-2,4-thiazolidinedione, the meaning of the HY is an acid whose acidic pK < 4, preferably hydrochloride acid, trifluoroacetic acid, formic acid, p-toluenesulfonic acid.

According to the process of our invention compounds of the general formula (II) are hydrogenated catalytically and the product is reacted with a compound affording X⁺ to give the compounds of the general formula (I) - where the X⁺ in the formula is alkali or alkaline-earth metals, preferably potassium, sodium, lithium, magnesium ions.

The advantage of the process according to our invention is that the amount of the catalyst can be significantly decreased in the hydrogenation of the new salts of the formula (II) in the presence of palladium-on-charcoal catalyst: 0.2g of 10% palladium-on-charcoal is needed for 1 g salt of the formula (II) [while in the EP 306228 3 g of 10% palladium-on-charcoal (150%) is used for 2 g base of the formula (III)]. After the reduction the catalyst is filtered off, the rosiglitazone base is isolated from the solution using a stoichiometric amount of a base.

An additional advantage of the process according to our invention is the high purity of the hydrogenated intermediate (HPLC purity: over 99.5%), which is further purified in the following last step.

| Synthesis of rosiglitazone base on the basis of our own experiments | | | | |
|---|---|---|---|---|
| Procedure | Ratio of catalyst (10% Pd/C) and substrate base of formula (III) | Yield (%) | Purity (HPLC,%) | Melting Point (°C) |
| EP 306228 | 150 % * | 75-80 | 96-97 | 153-155 |
| EP 306228 | 20 % | - | 66** | |
| According to the invention [starting from the trifluoroacetate salt of formula (II)] | 10-15 % | 79 | 99 | 153-155 |

| | | | | |
|---|---|---|---|---|
| * EP 306228, example 1 | | | | |
| ** measurement of the reaction mixture (isolation of the product was not possible): HPLC shows a conversion of 66%. | | | | |

The salt formation from the rosiglitazone base was carried out with equivalent amount of base. The alkali- and alkaline-earth salts of rosiglitazone were crystallised from a warm solution under mild cooling to give rosiglitazone salts, which could be filtered off easily and the products had a high purity. The potassium salt of rosiglitazone proved to be especially advantageous, because its purity was 99.9% (HPLC) and the individual impurity component amounted to max. 0.02 %.

As mentioned before the compounds according to our invention have valuable medicinal properties, therefore, the invention extends to those pharmaceutical products containing an active pharmaceutical ingredient of the general formula (I) or its tautomer form and/or its pharmaceutically acceptable solvate. These pharmaceutical products can be used for example to treat and/or prevent hyperglycaemia, hypertension, cardiovascular diseases and certain eating disorders.

According to our invention the therapeutical products are tablets and capsules etc. which are suitable for oral administration.

In accordance with the usual pharmaceutical practice we can use ingredients such as dilutors, filler materials, wetting agents, lubricants, colourants, flavourants or other usually applied components. We can typically employ carriers such as microcrystalline cellulose, starch, sodium starch glycollate, polyvinylpyrrolidone, polyvinylpolypyrrolidone, magnesium-stearate or sodium lauryl sulphate.

### HPLC-investigation of the reaction mixture during the synthesis of rosiglitazone and its purity

- Coloumn:: Lichrosphere RP8 5 µm 250 4mm
- Eluent:: A: 0.05 mol/l KH₂PO₄: acetonitrile = 95:5 pH=3.5
B: 0.05 mol/l KH₂PO₄: acetonitrile = 35:65 pH=3.5

| Gradient: | | | |
|---|---|---|---|
| Time(minutes) | A(%) | B(%) | Flowrate(ml/min) |
| 0 | 77 | 23 | 1 |
| 0.1 | 72 | 28 | 1 |
| 10.4 | 72 | 28 | 1 |
| 13.5 | 18 | 82 | 1 |
| 13.6 | 15 | 85 | 1 |
| 22.0 | 15 | 85 | 1 |
| 25.0 | 0 | 100 | 1 |
| 35.0 | 0 | 100 | 1 |

- Temperature:: 55°C
- Detection:: 215 nm UV
- Injected volume:: 20 µl
Sample preparation: samples are solved in acetonitrile : water : acetic acid 100:100:1 mixture or the reaction mixture is diluted in 35 times with this mixture.

The synthesis of the starting material of the formula (III) was reported by B.Cantello et al., [Bioorganic and Medicinal Letters, 4. 1181-4(1994)]:

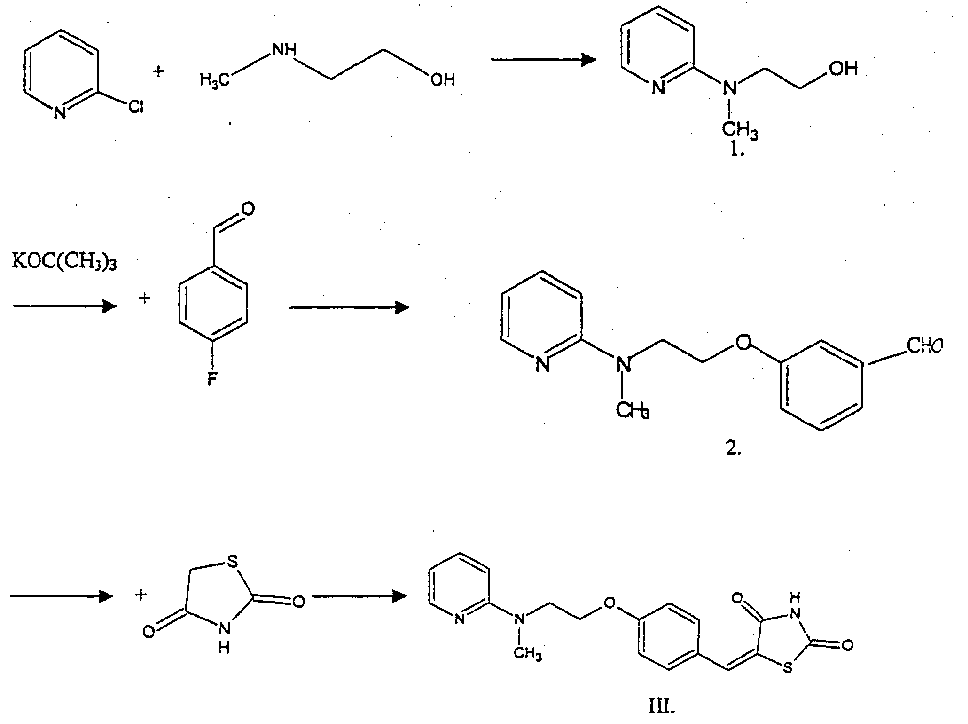

Based on the above publication the starting compound of the formula (III) could be prepared as follows:

### 1 Synthesis of 2-[N-methyl-N-(2-pyridyl)-amino]-ethanol

113.5 g (1 mol) of 2-chloro-pyridine and 300 g (4 mol) of 2-methylamino-ethanol was stirred for 15 hours at 150°C. The excess of 2-methylamino-ethanol was evaporated at 4 mbar, the residue was dissolved in 500 ml of dichloromethane and was extracted with 3 x 100 ml of water. The organic extract was dried (Na₂SO₄) and the solvent was evaporated. The title compound, 141.2 g (92.7%), was obtained in a purity of 99,5 % (HPLC). Boiling point: 90-94°C/4 mbar, n₂₀=1.5723.

### 2 Synthesis of 4-[2-(N-methyl-N-(2-pyridyl)-amino)-ethoxy)-benzaldehide

19.1 g (0.126 mol) of the intermediate 1 was dissolved in 100 ml of dimethyl-formamide and 14.4 g (0.126 mol) of potassium-tert-butoxide in 100 ml of dimethylformamide was added dropwise under nitrogen, then 15.6 g (0.126 mol) of 4-fluoro-benzaldehyde in 40 ml of dimethylformamide was added dropwise. The reaction mixture was stirred for 4 hours at 80°C and the solvent was evaporated. The oily residue was dissolved in 250 ml of toluene and extracted with 3 x 50 ml of water and 50 ml of brine. The solution contained 77 % of the title compound (HPLC). This solution was used directly in the next step.

### 3 Synthesis of 5-{4-[2-(N-methyl-N-(2-pyridyl)-amino)-ethoxy]-bezylidene}-thiazolidine-2,4-dione (III)

12.5 g (0.106 mol) of thiazolidine-2,4-dione and 1.4 g (0.01 mol) of piperidine acetate were added to the toluene solution of the compound 2 and it was heated under reflux for 4 hours then cooled to 10°C. The precipitated product was filtered off, washed with toluene and methanol. The yield of the title comound was 29.6 g (86%), HPLC: 94-95%. Melting point: 189-191°C.

The following Examples illustrate the invention:

### Example 1.

### 5-{4-[2-(N-methyl-N-(2-pyridyl)-amino)-ethoxy]-benzylidene}-2,4-thiazolidinedione trifuoroacetate (compound of formula II.: HY=CF₃COOH)

28.4 g (77 mmol) of crude 5-{4-[2-(N-methyl-N-(2-pyridyl)-amino)-ethoxy]-benzylidene}-2,4-thiazolidinedione was suspended in 500 ml of methanol and 6.8 ml of trifluoroacetic acid was added dropwise during stirring. Heating the reaction mixture to boiling a solution was formed to give the crystalline product precipitating under cooling. The yield of the title product was 34 g (90.4%), the melting point 182-184°C. HPLC: 99.5%. The trifluoroacetate salt was treated with a stoichiometric amount of sodium-hydoxide to give the title compound as a base with a the melting point 194-196°C.

| **Other salts of formula (II):** | | **HPLC** |
|---|---|---|
| **HCl-salt** | melting point: 235-238°C | 96 % |
| **Tosylate-salt** | melting point: 209-211°C | 95 % |
| **Formiate-salt** | melting point: 195-199°C | 96 % |

### Example 2.

### Potassium salt of rosiglitazone (compound of formula I.: X⁺=K⁺)

9.4 g (20 mmol) of trifluoroacetate salt of 5-{4-[2-(N-methyl-N-(2-pyridyl))-amino-ethoxy]-benzylidene}-2,4-thiazolidinedione was dissolved in 150 ml of acetic acid and was hydrogenated under pressure (5 bar) at 70°C in the presence of 15 ml aqueous suspension of 1.5 g 10% palladium-on-charcoal. After the hydrogenation the reaction mixture was filtered and the solvent was evaporated. The residue was dissolved in a 2:1 mixture of methanol:water and 3.6 g (20 mmol) of 5.6 N potassium hydroxide solution was added. The precipitated crystalline product was filtered and was washed with water and methanol. 5.6 g (79%) rosiglitazone base was obtained with a melting point of 153-155°C. HPLC: 99 %.
5.35 g (15 mmol) of the rosiglitazone base was suspended in 100 ml of ethanol and 2.7 ml (15 mmol) of 5.6 N potassium hydroxide was added dropwise during stirring. The suspension was warmed up to 70°C giving a solution, whose cooling afforded the crystalline product. It was stirred for 4 hours at 0°C then was filtered and washed with 0°C ethanol. 5.04 g (85%) of the rosiglitazone potassium salt was obtained with a melting point of 203-205°C. HPLC: > 99.9%.

### Example 3.

### Sodium salt of rosiglitazone (compound of formula I.: X⁺=Na⁺)

0.71 g (2 mmol) of rosiglitazone base was suspended in 10 ml of ethanol. 0.53 ml (2 mmol) of 3.8 N sodium hydroxide was added and was stirred for 5 hours at 20°C. The product was filtered and was washed with ethanol Yield: 0.64 g (84.4%) of the title compound, melting point: 258-261°C. HPLC purity: 99.6%.

### Example 4.

### Lithium salt of rosiglitazone (compound of formula I.: X⁺=Li⁺)

0.71 g (2 mmol) of rosiglitazone base was suspended in 10 ml of ethanol, 84 mg of lithium hydroxide monohydrate was added. The reaction mixture warmed up to 60°C giving a solution from what the product was crystallised from diethyl ether. Yield: 0.65 g (89.5%) of the title product, melting point: 138-141°C.

### Example 5.

### Magnesium salt tetrahydrate of rosiglitazone (compound of formula I.:X⁺=0.5 Mg²⁺)

1.97 g (5 mmol) of potassium salt of rosiglitazone, described in Example 1., was dissolved in 20 ml of water, 0.4 g of magnesium sulphate was added to it and was stirred for 5 hours at 20°C. The precipitated product was filtered and was washed with water. The yield of the title compound was 1.81 g (82%). HPLC purity: 99.7%.

### Example 6.

### 4 mg tablet of rosiglitazone-potassium salt

The tablet contains 4 mg of 5-{4-[2-(N-methyl-N-(2-pyridyl)-amino)-ethoxy]-benzyl}-2,4-thiazolidinedione potassium salt, 100 mg of lactose monohydrate, 30 mg of microcrystalline cellulose, 10 mg of starch and 0.5 mg of magnesium stearate.

The following pages 9 and 10 of the description refer to preferred embodiments of the invention.
1.New process for synthesis of the compounds of the general formula (I) or their tautomer forms and their solvates - where the meaning of X⁺ in the formula is alkali or alkaline-earth metal ion - characterised by catalytic hydrogenation of the compounds of general formula (II), whose product is reacted with a compound affording X⁺ to give a compound of formula (I). 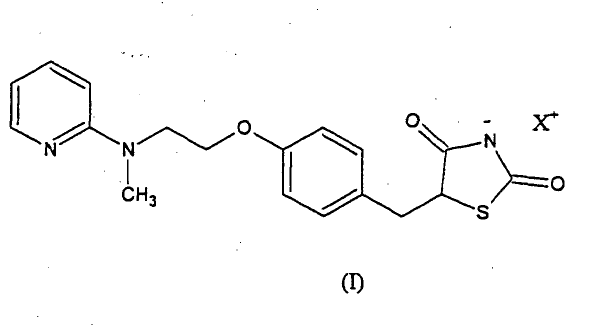
2. The compounds of the general formula (I) or their tautomer forms where the meaning of X⁺ in the formula is alkali or alkaline-earth metal ion.
3. The compounds in claim 2 characterised by the meaning of X⁺ in the formula (I) where it is potassium ion.
4. The compounds in claim 2 characterised by the meaning of X⁺ in the formula (I) where it is sodium ion.
5. The compounds in claim 2 characterised by the meaning of X⁺ in the formula (I) where it is lithium ion.
6. The compounds in claim 2 characterised by the meaning of X⁺ in the formula (I) where it is magnesium ion.
7. The compounds of general formula (II) or their tautomer forms where the name of the base component in the formula is: 5-{-4-[2-(N-methyl-N-(2-pyridyl)-amino)-ethoxy]-benzylidene}-2,4-thiazolidinedione, the meaning of the HY is an acid whose acidic pK < 4. 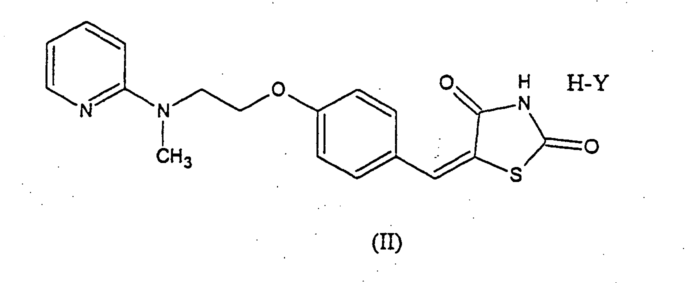
8. The compounds in claim 7 characterised by the meaning of HY where it is hydrochloride acid, p-toluenesulfonic acid, formic acid or trifluoroacetic acid.
9. The pharmaceutical products characterised by containing any of the compound of the formula (I) or its tautomer form and/or its pharmaceutically accepted solvate as active pharmaceutical ingredient together with the usually applied carriers and/or accessory materials of the pharmaceutical technology.
10. The use of medicaments employing the compounds of the general formula (I), their tautomer forms and/or their pharmaceutically acceptable solvates for the treatment or/and preventing hyperglycaemia, hypertension, cardiovascular diseases and certain eating disorders.

## Claims

1. The compound of general formula (I) or their tautomer forms where the meaning of X⁺ in the formula is potassium ion.

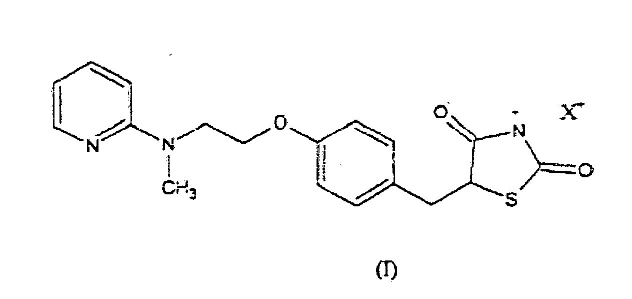

2. The pharmaceutical products **characterized by** containing the compound according to claim 1 or its tautomer form and/or its pharmaceutically accepted solvate as active pharmaceutical ingredient together with the usually applied carriers and/or accessory materials of the pharmaceutical technology.

3. Use of a compound according to claim 1, their tautomer forms and/or their pharmaceutically acceptable solvates for the preparation of a medicament for the treatment and/or prevention of hyperglycaemia, hypertension, cardiovascular diseases and certain eating disorders.
